# EUROPEAN PATENT SPECIFICATION

(11) **EP 1 217 005 B1**
(45) Date of publication and mention of the grant of the patent: **30.06.2004**
(21) Application number: 01129698.5
(22) Date of filing: 13.12.2001
(51) Int. Cl.: C07K 14/335, C07K 7/02, A61K 38/08

(54) **Synthetic peptide having antitumor cytotoxic activity**
Synthetisches peptid mit antitumoralen cytotoxischen Wirkung
Peptide synthétique avec activité antitumorale cytotoxique

(30) Priority: 22.12.2000 IT MI002804
(43) Date of publication of application: 26.06.2002
(73) Proprietor: Fichera, Giuseppe, 95125 Catania (IT)
(72) Inventor: Fichera, Giuseppe, 95125 Catania (IT)
(74) Representative: Gervasi, Gemma, Dr.

(56) References cited:
- EP-A- 0 545 352
- BILLOT-KLEIN, DANIELE ET AL: "Peptidoglycan structure of Enterococcus faecium expressing vancomycin resistance of the VanB type" BIOCHEM. J. (1996), 313(3), 711-15 , 1996, XP002195578

## Description

### PRIOR ART

The studies published relating to substances suitable for the treatment of the tumor pathologies are very numerous.

Among these ones some studies about researches which proved the antitumor activity of Lactobacillus casei towards experimental and human malignant tumors have been published.

Results of these studies have been published for example by Kato I., Kobayashi S., Yokokura T., Mutai M. (1981): Antitumor Activity of Lactobacillus casei in Mice, Gann 72:517 and by Matsuzaki T., Yokokura T., Mutai M. (1988): Antitumor Effect of Intrapleural Administration of Lactobacillus casei in Mice, Cancer Immunol. Immunother. 26:209 and, by Matsuzaky T., Yokokura T., Mutai M. (1988): The Role of Lymph Node Cells in the Inhibition of Metastasis by Subcutaneous Injection of Lactobacillus casei in Mice, Med. Microbiol. Immunol. 177:245, by Nanno M., Shimizu-Takeda T., Milke A., et al. (1989): Increased Production of Cytotoxic Macrophage Progenitors by Lactobacillus casei in Mice, J. Leukoc. Biol. 46:89.

Moreover it has been proved that by feeding with fermentation products with Lactobacillus casei an antitumor effect is obtained as described for example by Friend BA, Farmer RE, Shahani KM (1982): Effect of Feeding and Intraperitoneal Implantation of Yoghurt Culture Cells on Ehrlich Ascites Tumor, Milchwissenschaft 37:708.

The antitumor effect of Lactobacillus casei has been exclusively ascribed to an immunostimulation action because a direct toxicity towards tumor cells in vitro has not been found, as proved for example by Kato I. et al. (I.c.).

Pharmaceutical compositions containing the peptidoglycan obtained by mechanical treatment of the Lactobacillus casei cells followed by treatment with trichloroacetic acid and solubilization in water (EP 0545352 A1) have also been patented.

However the antitumor activity of compositions derived by Lactobacillus casei is rather low.

### SUMMARY

Now a synthetic peptide which shows a high inhibition activity in vitro of tumor cell lines with direct cytotoxic mechanism has been found.

Said peptide has the following chemical structure:

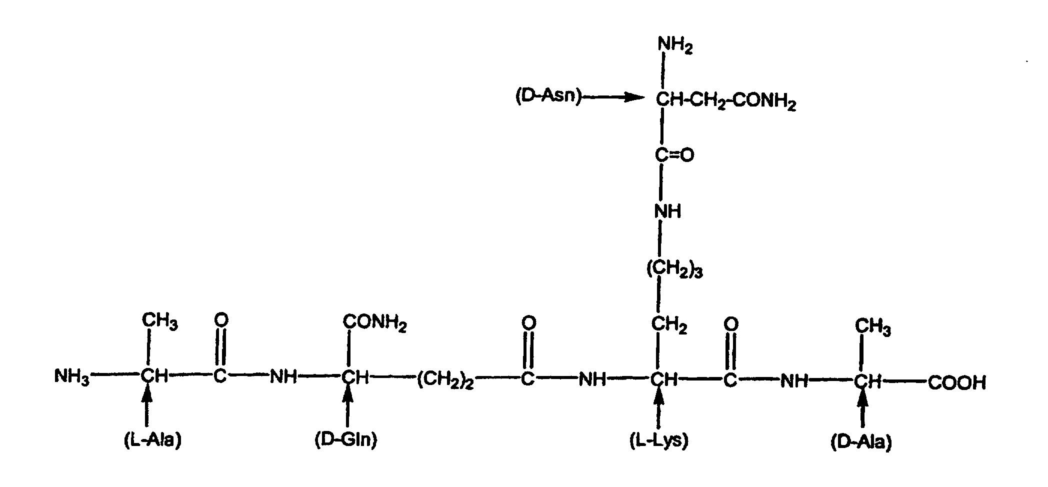

It may be used for the preparation of pharmaceutical compositions suitable for the treatment of tumor pathologies.

Therefore the present invention concerns said peptide and the related pharmaceutical compositions suitable for the antitumor therapy.

### DETAILED DESCRIPTION OF THE INVENTION

The present invention refers to a synthetic peptide having an inhibition activity of tumor cell lines in vitro and to the related pharmaceutical compositions suitable for the antitumor therapy.

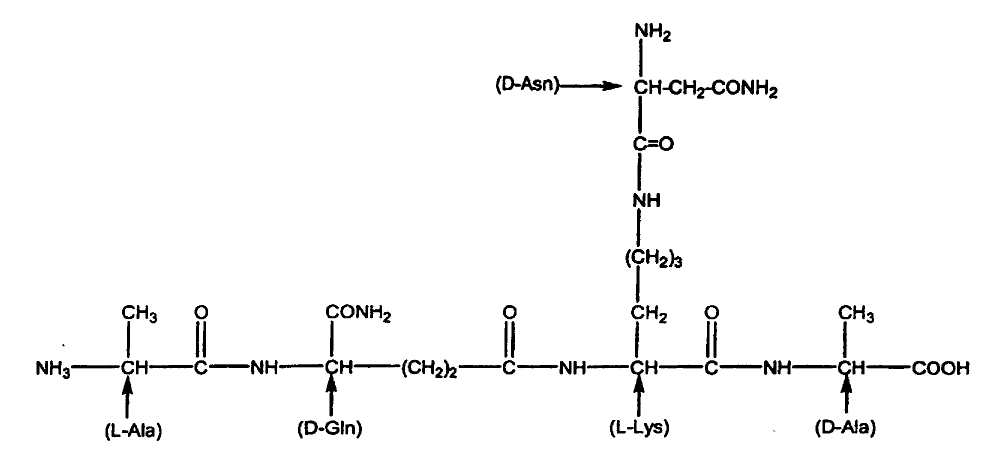

Said peptide has the following chemical structure:

Said peptide comprises five aminoacids. Three of these aminoacids have dextrorotatory (-D) configuration and the presence of these aminoacids is responsible of the direct cytotoxic activity towards the tumor cells.

In fact compositions having the same chemical structure, but with all the aminoacids in levorotatory (-L) configuration do not point out any cytotoxic effect

Moreover it is important to notice that the cytotoxic activity is carried out only on the tumor cells while the normal cells are not interested by this activity.

On the contrary, in the normal cells an increase of the metabolic activity which has been proved particularly, besides on cultures of normal human fibroblasts, also on normal human lymphocytes.

The peptide according to the invention is produced according to the conventional method of synthesis in solid phase. The synthesis has been carried out by: Eurogentec-Parc Scientifique du Sart Tilman, 4102 Seraing-Belgium.

The peptide obtained after lyophilization shows a white powder aspect having a high solubility in water.

In Table 1 the chemical and chemico-physical characteristics of the peptide according to the invention are reported.

**TABLE 1**

| | |
|---|---|
| - Molecular Weight | 529.56 dalton |
| - Length | 5 aminoacid residuals |
| - 1 microgram | 1888.349 pMoles |
| - Molar Extinction Coefficient | 0 ± 5% |
| - Absorbance (280) | CYW absent |
| - Isoelectric Point | 9.00 |
| - Charge at pH 7 | 0.91 |

In Table 2 the complete analysis of the peptide according to the invention is reported.

**TABLE 2**

| Aminoacids | Numerical Counting | % by weight | % frequency |
|---|---|---|---|
| Load (RKHYCDE) | 1 | 24.20 | 20.00 |
| Acid (DE) | 0 | 0.00 | 0.00 |
| Basic (KR) | 1 | 24.20 | 20.00 |
| Polar (NCQSTY) | 2 | 45.74 | 40.00 |
| Hydrophobic (AILFWV) | 2 | 26.84 | 40.00 |
| A Ala | 2 | 26.84 | 40.00 |
| C Cys | 0 | 0.00 | 0.00 |
| D Asp | 0 | 0.00 | 0.00 |
| E Glu | 0 | 0.00 | 0.00 |
| F Phe | 0 | 0.00 | 0.00 |
| G Gly | 0 | 0.00 | 0.00 |
| H His | 0 | 0.00 | 0.00 |
| I Ile | 0 | 0.00 | 0.00 |
| K Lys | 1 | 24.20 | 20.00 |
| L Leu | 0 | 0.00 | 0.00 |
| M Met | 0 | 0.00 | 0.00 |
| N Asn | 1 | 21.55 | 20.00 |
| P Pro | 0 | 0.00 | 0.00 |
| Q Glu | 1 | 24.20 | 20.00 |
| R Arg | 0 | 0.00 | 0.00 |
| S Ser | 0 | 0.00 | 0.00 |
| T Thr | 0 | 0.00 | 0.00 |
| V Val | 0 | 0.00 | 0.00 |
| W Trp | 0 | 0.00 | 0.00 |
| Y Tyr | 0 | 0.00 | 0.00 |
| B Asx | 0 | 0.00 | 0.00 |
| Z Glx | 0 | 0.00 | 0.00 |
| X Xxx | 0 | 0.00 | 0.00 |
| .Ter | 0 | 0.00 | 0.00 |

### BIOLOGICAL ACTIVITY TEST

The quantitative evaluation of the effect of the peptide according to the present invention on the cell proliferation and vitality is carried out by the colorimetric method based on the reduction of the tetrazolium salts (MTT or XTT) to formazan.

As the conversion to formazan occurs by action of the succino-dehydrogenase enzyme only in the living and metabolically efficient cells, the amount of formazan is proportional to the number of the living cells.

The test is carried out in a plate with 96 wells.

To 100 µl of cell suspension (25,000 cells/100 µl) in RPMI 1640 medium without phenol red (RP) with 10% of calf fetal serum, distributed in the wells, the amount of peptide to test (10 µg) is added. The plate is then incubated for 3 hours in thermostat at 37 °C and in atmosphere at 5% CO₂. At the end of this period to each well 50 µl of revealing mixture consisting of a) a solution of XTT (sodium 3'-[1-(phenylaminocarbonyl)-3,4tetrazolium]-bis(4-methoxy-6-nitro) benzene sulfonic acid hydrate) in RPMI 1640, 1 mg/ml without phenol red, sterile; and b) a solution in PBS (phosphate buffered saline) containing 0.383 mg/ml of PMS (N-methyl dibenzopyrazine methyl sulfate) are added. The mixture, created a little before the addition to the cell suspension, is prepared mixing 5 ml of a) with 0.1 ml of b). Of this mixture 50 µl for each well are added. It is left to incubate for 18 hours at 37 °C and at 5% CO₂ and then it is read at the spectrophotometer for plates [microtiter plate (ELISA) reader]. The wavelength to measure the absorbance of the produced formazan ranges from 450 to 500 nm; the reference wavelength must be greater than 650 nm. The estimation of the formazane produced as expression of cell vitality is compared with that one of the control wells (cell suspension without peptide) and then the percentage of vitality of the cells tested with the peptide is calculated.

This test of succino-dehydrogenasic activity as expression of the cell vitality has been carried out on several tumor cell lines as one deduces from Table 3.

**TABLE 3**

| % Decrease of the Succino-dehydrogenasic Activity by effect of the Peptide (10 µg / 100 µl) | |
|---|---|
| Cell line | |
| K562 | 19.2 ± 5.3 |
| YAC-1 | 31.5 ± 4.7 |
| EATC | 21.2 ± 3 |
| Mamm. Carc. | 18.6 ± 5.1 |
| C6 (glioma) | 23.22 ± 3.0 |
| Neuroblastoma | 19.81 ± 2.4 |

The above reported test has been repeated at different concentrations of YAC-1 cells (from 12,500 / 10 µl to 50,000 / 100 µl) and at different concentrations of peptide (from 1 µg /100 µl to 10 µg / 100 µl).

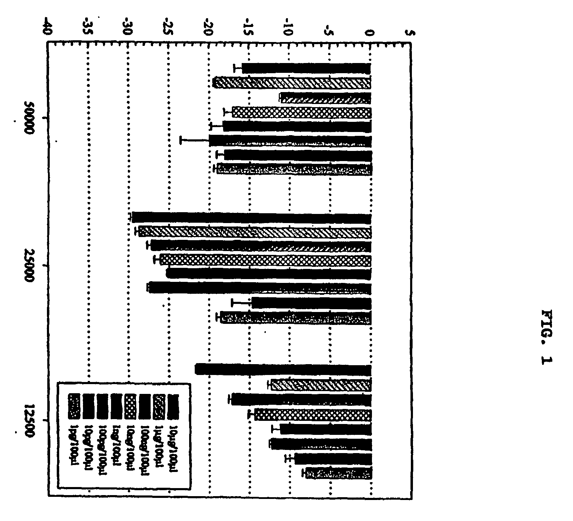

The results are reported in Figure 1 wherein in the ordinate the trend of the succino-dehydrogenasic activity expressed as percent variation is represented and in the abscissa the concentration of the YAC-1 cells is represented. The concentration of the peptide is reported in the internal frame.

As clearly appears from Fig. 1, the peptide according to the present invention has a marked cytotoxic activity to the concentration of 10 pg/ 100 µl of tumor cell suspension at several cell concentrations and precisely 12,500, 25,000 and 50,000/100 µl. The decrease in the succino dehydrogenasic activity to a maximum of - 30% finds explanation in that the tumor cells are sensible to the action of the peptide only in a determined phase of the replicative cycle and precisely in the G2/M phase. Therefore in an asynchronous cell population only the cells which are in the above mentioned replicative phase are stopped in their activity. Consequently only a certain percentage and not the totality of the cells shows the inactivation of the enzymatic system expression of the intramitochondrial metabolic disease. It has been proved during the research that a tumor cell population synchronised with Hydroxyurea, brought into contact with the peptide and followed in time along the various multiplicative phases, shows an evident increase of mortality in relation to the G2/M phase reaching a decrease even of 70% in the succino-dehydrogenasic activity.

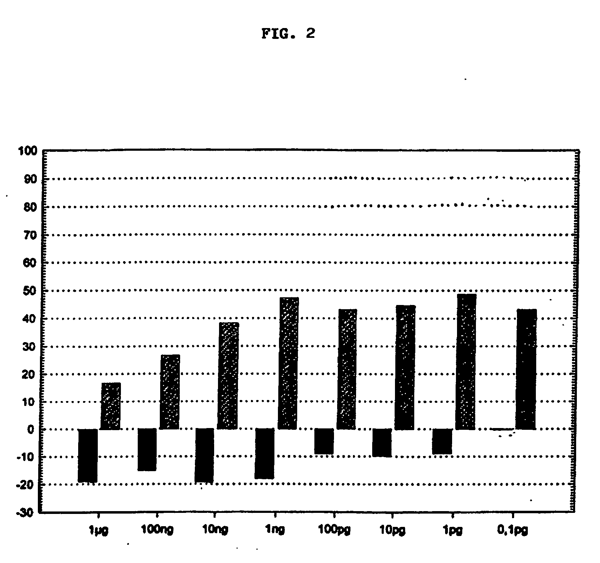

It has moreover verified, during the research, that the peptide according to the present invention binds with hexokinase II (glucose-6-Phosphotranspherase) first enzyme of the glycolytic chain which is localized on the external mitochondrial membrane. The Fig. 2 shows the in vitro response of the tumor cells of murine lymphoma (YAC-1) cultured together with the peptide with addition of hexokinase II (dashed graphs) and without addition of hexokinase II (black graphs).

The test has been carried out in the following conditions:
- peptide solubilized with a 0.1 % solution of acetonitrile in H₂O;
- hexokinase II: 1.73 U/test;
- YAC-1 cells (250,000/ml). In each test: 90 µl.

In Figure 2 in the ordinate the percentage of succino-dehydrogenasic activity is represented and in the abscissa the amount of added peptide is represented.

It is known that in the tumor cells the hexokinase II activity is to 20 times stronger than the normal cells. Hexokinase II is linked to the external mitochondrial compartment in a receptorial site adjacent to a mitochondrial purine and near to the insulin site. Then it is evident that the increased bond of the hexokinase II to the purine of the external mitochondrial membrane not only accelerates glycolysis giving hexokinase II a better access to mitochondrial ATP (adenosine triphosphate), but also stimulates the tricarboxylic acids cycle supplying the mithocondrion with ADP (adenosine diphosphate) which acts as an acceptor for the phosphoric groups.

Thus the glucose metabolism and the production of some aminoacids, as intermediate products of the tricarboxylic cycle, are increased and the request for a stronger replicative activity is satisfied.

The peptide according to the present invention blocking the hexokinase II activity determines a primary stop of glycolysis with subsequent block of the metabolic chain and of the replicative activity of the tumor cells.

Thanks to the above reported characteristics the peptide according to the present invention may be used for the preparation of pharmaceutical compositions suitable for the therapy of the human tumor pathologies.

Said compositions contain pharmacologically effective amounts of said peptide in mixture with diluents or excipients normally used in the pharmaceutical technique.

Preferably said peptide is dissolved in buffered aqueous solution, and if necessary it is formulated with liposomes consisting of derivatives of cholesterol, phosphatidylcholine and phosphatidylinositol.

Finally the present invention also refers to a therapeutic method for the treatment of human tumor pathologies comprising the administration of an effective dose of the peptide according to the present invention.

## Claims

1. Synthetic peptide having the following chemical structure: 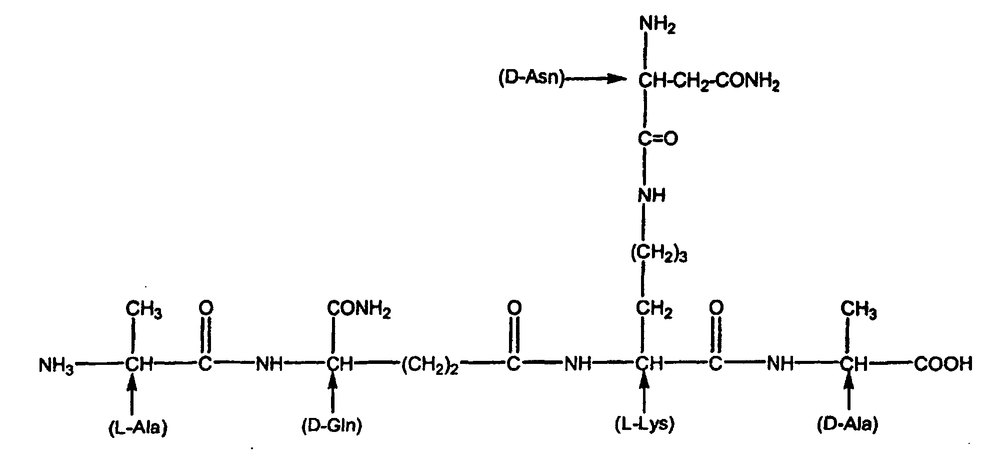 having an in vitro inhibition activity of tumor cell lines.

2. Peptide as claimed in claim 1, **characterized in that** it has a molecular weight equal to 529.56 dalton and isoelectric point of 9.00.

3. Peptide as claimed in claim 1, **characterized in that** it has the following composition by weight: 26.84% alanine, 24.20% lysine, 21.55% asparagine and 24.20% glutamine.

4. Use of the peptide as claimed in claim 1 for the preparation of pharmaceutical compositions suitable to the therapy of the human tumor pathologies.

5. Pharmaceutical compositions comprising the peptide as claimed in claim 1, suitable for the therapy of the human tumor pathologies.

6. Compositions as claimed in claim 5, **characterized in that** they contain pharmacologically effective amounts of said peptide in mixture with diluents or excipients normally used in the pharmaceutical technique.

7. Compositions as claimed in claim 5, **characterized in that** said peptide dissolved in buffered aqueous solution is formulated with liposomes.

## Patentansprüche

1. Synthetisches Peptid, mit der folgenden chemischen Struktur: 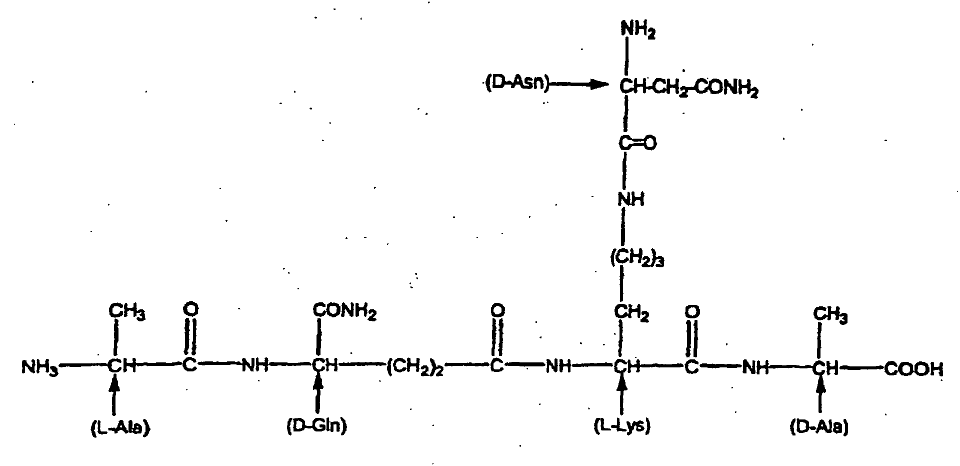 welches eine in vitro Hemmungsaktivität von Tumorzelllinien aufweist.

2. Peptid nach Anspruch 1, **dadurch gekennzeichnet, dass** es ein Molekulargewicht gleich 529.56 Dalton und einen isloelektrischen Punkt von 9.00 aufweist.

3. Peptid nach Anspruch 1, **dadurch gekennzeichnet, dass** es die folgende Gewichtszusammensetzung aufweist: 26.84% Alanin, 24.20% Lysin, 21.55% Asparagin und 24.20% Glutamin.

4. Verwendung des Peptids nach Anspruch 1 für die Herstellung pharmazeutischer Zusammensetzungen, welche geeignet zur Therapie menschlicher Tumorpathologien sind.

5. Pharmazeutische Zusammensetzungen, beinhaltend das Peptid nach Anspruch 1, welche geeignet zur Therapie menschlicher Tumorpathologien sind.

6. Zusammensetzungen nach Anspruch 5, **dadurch gekennzeichnet, dass** sie pharmakologisch wirksame Mengen des Peptids enthalten in Mischung mit Verdünnungsmitteln oder Arzneiträgern, die normalerweise in pharmazeutischen Verfahren verwendet werden.

7. Zusammensetzungen nach Anspruch 5, **dadurch gekennzeichnet, dass** das Peptid aufgelöst in gepufferter wässriger Lösung mit Liposomen formuliert ist.

## Revendications

1. Peptide synthétique ayant la structure chimique suivante : 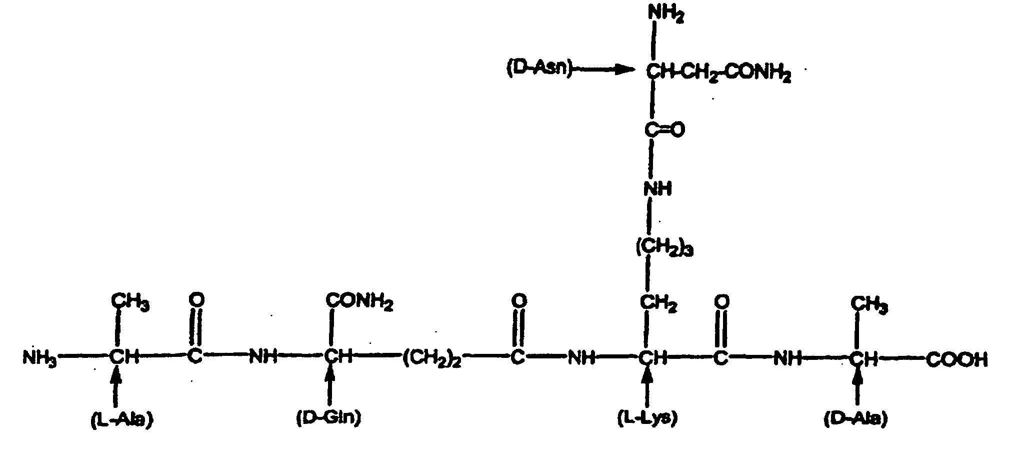 ayant une activité d'inhibition in vitro des lignées de cellules de tumeur.

2. Peptide selon la revendication 1, **caractérisé en ce qu'**il a un poids moléculaire égal à 529,56 Dalton et un poids isoélectrique de 9,00.

3. Peptide selon la revendication 1, **caractérisé en ce qu'**il a la composition suivante en poids : 26,84% d'alanine, 24,20% de lysine, 21,55% d'asparagine et 24,20% de glutamine.

4. Utilisation du peptide selon la revendication 1 pour la préparation de compositions pharmaceutiques appropriées à la thérapie des pathologies des tumeurs humaines.

5. Compositions pharmaceutiques comprenant le peptide selon la revendication 1, appropriées pour la thérapie des pathologies de tumeurs humaines.

6. Compositions selon la revendication 5, **caractérisées en ce qu'**elles contiennent des quantités pharmacologiquement efficaces dudit peptide en mélange avec des diluants ou excipients normalement utilisés en technique pharmaceutique.

7. Compositions selon la revendication 5, **caractérisées en ce que** ledit peptide dissous dans une solution aqueuse tamponnée est formulé avec des liposomes.
